# EUROPÄISCHE PATENTSCHRIFT

(11) **EP 1 946 702 B1**
(45) Veröffentlichungstag und Bekanntmachung des Hinweises auf die Patenterteilung: **07.03.2012**
(21) Anmeldenummer: 07001290.1
(22) Anmeldetag: 22.01.2007
(51) Int. Cl.: A61B 6/00, A61B 19/00

(54) **Abbildung anatomischer Strukturen**
Illustration of anatomic structure
Représentation de structures anatomiques

(43) Veröffentlichungstag der Anmeldung: 23.07.2008
(73) Patentinhaber: BrainLAB AG, 85622 Feldkirchen (DE)
(72) Erfinder: Tuma, Gregor, 81675 München (DE); Wohlgemuth, Richard, 83646 Bad Tölz (DE)
(74) Vertreter: Engelhard, Maximilian

(56) Entgegenhaltungen:
- EP-A- 1 693 798
- US-A1- 2005 192 495
- US-B1- 6 195 409

## Beschreibung

Die vorliegende Erfindung betrifft ein Verfahren und eine entsprechende Vorrichtung zum Erfassen eines spezifischen Teils einer anatomischen Struktur eines Menschen oder Tieres mittels einer Abfolge von Abbildungen. Insbesondere sollen Abbildungsbedingungen von Abbildung zu Abbildung in geeigneter Weise geändert werden, um Information über das spezifische Teil in ausreichendem Umfang zu erzielen.

Werden bisher Abbildungen von einem Patienten gemacht, so liegt dieser beispielsweise auf einer Liege und ein Abbildungsgerät, zum Beispiel ein Röntgengerät ist in einer bestimmten Lage zu der Liege angeordnet. Ein spezifischer Teil der anatomischen Struktur des Patienten soll mit dem Röntgengerät abgebildet werden, beispielsweise das Becken. Häufig wird mit der ersten Abbildung nicht der spezifische Teil erfasst. Der Arzt korrigiert dann die Lage des Röntgenapparats. Danach wird eine zweite Abbildung getätigt. Erfasst die zweite Abbildung wiederum nicht das spezifische Teil, so ist noch eine weitere Abbildung notwendig.

US 2005/192495 A1 zeigt einen medizinischen Untersuchungsapparat zum vereinfachten Vergleich von medizinischen Bildern. Dazu wird ein Beobachtungswinkel, unter dem eine Prothese betrachtet wird, abgeschätzt, wobei ein Beobachtungswinkel, unter dem ein 3D-Modell der Prothese betrachtet wird, vorbekannt ist. Das 3D-Modell wird somit als vorbekannt angenommen.

Aufgabe der Erfindung ist es, ein Verfahren und eine Vorrichtung bereit zu stellen, die es erleichtert, ein spezifisches Teil einer anatomischen Struktur eines Patienten zu erfassen.

Vorstehende Aufgabe wird durch die Gegenstände der unabhängigen Ansprüche gelöst. Vorteilhafte Weiterbildungen gehen aus den abhängigen Ansprüchen hervor.

Vorzugsweise wird eine Abbildung erstellt, mit der ein Teil der anatomischen Struktur abgebildet wird. Dieser Teil wird im Folgenden "abgebildeter Teil" genannt. Der abgebildete Teil kann mit dem spezifischen Teil übereinstimmen. Insbesondere bei der ersten Abbildung kann dies jedoch häufig nicht der Fall sein, wie oben ausgeführt wurde. Die Abbildung erfolgt unter bestimmten Abbildungsbedingungen, wie beispielsweise die Richtung, aus der die Abbildung erfolgt ("Abbildungsrichtung" genannt). Weiter wird bei der Abbildung ein gewisser Bereich erfasst, der abgebildet wird. Dieser Bereich wird Abbildungsbereich genannt und ist ein weiteres Beispiel für eine Abbildungsbedingung. Der Abbildungsbereich wird beispielsweise durch die Abbildungsoptik, den Abstand zwischen dem Abbildungsgerät und dem abgebildeten Teil und/oder durch Form und Größe des Abbildungssensors bestimmt. Der Abbildungsbereich ist insbesondere der Raum, aus dem die Abbildungssignale von dem Abbildungsgerät detektiert werden. Die Abbildungsbedingungen umfassen oder bestimmen also insbesondere die Abbildungsrichtung und/oder den Abbildungsbereich.

Beispiele für Abbildungsgeräte sind Röntgengeräte, insbesondere CT-Geräte, Kemspintomographen und Ultraschallgeräte. Abbildungen umfassen also insbesondere Röntgenaufnahmen, Kernspinaufnahmen und Ultraschallaufnahmen.

Vorzugsweise werden dem Verfahren Informationen über das spezifische Teil bereitgestellt, die dieses insbesondere kennzeichnen, beschreiben und/oder identifizieren. Vorzugsweise wird eine Datenbank, die Informationen über die anatomische Struktur enthält, basierend auf der Abbildung abgefragt. Die Datenbank kann allgemeine Informationen über für einen Menschen typische anatomische Strukturen enthalten und/oder für den zu untersuchenden Patienten spezifische Informationen. Die Datenbank umfasst vorzugsweise Informationen über den abgebildeten Teil und den spezifischen Teil, insbesondere ihre relative Lage zueinander, wie sie typischerweise beim Menschen oder bei bestimmten Patiententypen gegeben sind und/oder spezifische Informationen über die relative Lage bei dem zu untersuchenden Patienten, von dem der spezifische Teil erfasst werden soll.

Die Abfrage der Datenbank erlaubt die Bestimmung zumindest eines Teils der Abbildungsbedingungen. Beispielsweise kann durch einen Abgleich mit den Daten der Datenbank über die anatomische Struktur ermittelt werden, wie groß der erfasste Ausschnitt aus der anatomischen Struktur ist. Beispielsweise kann aus der relativen Lage von Landmarken oder markanten Linien und der Form der Linien oder Umrisse von Knochenstrukturen erkannt werden, aus welcher Richtung die Abbildung erfolgte. Diese Landmarken und Linien können beispielsweise durch Mustererkennungsalgorithmen und/oder Kontrasterhöhungsverfahren automatisch extrahiert und mit entsprechenden Daten der Datenbank verglichen werden.

Die bestimmten Abbildungsbedingungen können beispielsweise angezeigt oder abgespeichert werden, so dass ein Arzt hierauf basierend über eine Änderung der Abbildungsbedingungen entscheiden kann. Insbesondere kann er entscheiden, ob er es bei der erfolgten Abbildung belässt oder eine weitere Abbildung durchführt. Vorzugsweise wird erfindungsgemäß insbesondere automatisch durch eine Vorrichtung und/oder ein Programm eine Änderung der Abbildungsbedingungen (oder zumindest eines Teils davon) durchgeführt. Eine Änderung der Abbildungsbedingungen erfolgt insbesondere dann, und insbesondere automatisch, wenn bei einem Abgleich der Abbildung, also ein Vergleich der Abbildung mit den in der Datenbank gespeicherten Daten bestimmt wird, dass der abgebildete Teil nicht mit dem spezifischen Teil übereinstimmt. Vorzugsweise werden dem Verfahren Informationen über den spezifischen Teil bereitgestellt, beispielsweise durch Benutzereingabe, die eine Identifikation des spezifischen Teils in der Datenbank erlauben. Diese Informationen über das spezifische Teil umfassen insbesondere Angaben, die es erlauben, die Lage des spezifischen Teils in der anatomischen Struktur zu bestimmen und/oder Angaben, die die Größe des zu erfassenden spezifischen Teils bestimmen. Beispielsweise wird durch die Eingabe des Begriffs "Becken" die Lage und die Größe des spezifischen Teils bestimmt. Vorzugsweise umfassen Informationen auch die gewünschten Abbildungsbedingungen, zum Beispiel die Richtung, aus der eine Abbildung des spezifischen Teils zu erfolgen hat, also Informationen über die Orientierung des spezifischen Teils relativ zum Abbildungsgerät. Vorzugsweise werden die bestimmten Abbildungsbedingungen mit den Informationen über das spezifische Teil verglichen und basierend auf den Informationen der Datenbank erfolgt dann eine Änderung der Abbildungsbedingungen für eine weitere Abbildung, so dass mit den neuen Abbildungsbedingungen das spezifische Teil erfasst wird, insbesondere in der gewünschten Größe und/oder Auflösung erfasst wird und insbesondere mit der passenden Orientierung, das heißt aus der korrekten Abbildungsrichtung.

Vorzugsweise werden Informationen zum Auffinden des spezifischen Teils in der Datenbank bereitgestellt, wie zum Beispiel Begriffe, die das spezifische Teil identifizieren, also zum Beispiel anatomische Begriffe wie Acetabulum, Schenkelhals usw. Außerdem kann die Ausdehnung oder Größe des spezifischen Teils angegeben werden. Beispielsweise kann angegeben werden HWS (Halswirbelsäule) von C1 bis C7.

Mit Hilfe der Datenbank kann die relative Lage und/oder die Ausdehnung des spezifischen Teils relativ zum abgebildeten Teil und damit auch relativ zum Abbildungsbereich bestimmt werden. Ist diese relative Lage und/oder die Ausdehnung bekannt, so kann der Abbildungsbereich mittels des Abbildungsgeräts geändert werden. Beispielsweise kann das Abbildungsgerät, insbesondere dessen für die Abbildung empfindlicher Bereich verfahren werden, der Abstand geändert werden oder die Abbildungsoptik geändert werden. Insbesondere kann der Ausschnitt geändert werden. Durch die vorgenannten Änderungen kann erreicht werden, dass ein Abbildungsbereich und eine Abbildungsrichtung bei einer nächsten Aufnahme für die Erfassung des spezifischen Teils passend sind. Dies bedeutet, dass das spezifische Teil im Abbildungsbereich dann enthalten ist und insbesondere aus einer gewünschten Richtung aufgenommen wird. Basierend auf der bestimmten relativen Lage und/oder der Ausdehnung können also die Abbildungsbedingungen oder zumindest ein Teil davon geändert werden, um mit der nächsten Aufnahme das spezifische Teil zu erfassen. Bei den vorgenannten Ausführungen kann der Begriff "relative Lage" auch eine relative Orientierung des abgebildeten Teils relativ zum spezifischen Teil umfassen, also eine Beziehung zwischen der Aufnahmerichtung des abgebildeten Teils und einer gewünschten Aufnahmerichtung für das spezifische Teil.

Wie bereits oben ausgeführt wurde, kann neben einer Identifikation des spezifischen Teils oder einer Beschreibung des spezifischen Teils auch zusätzliche Information dem Verfahren bereitgestellt werden, die die Art der Abbildung beschreiben, also beispielsweise eine gewünschte Abbildungsbedingung, wie zum Beispiel eine gewünschte Aufnahmerichtung und/oder Abbildungsbereich bei der Abbildung des spezifischen Teils. Eine derartige Information wird hierin "Art-Information" bezeichnet. Beispiele hierfür wären eine Aufnahme aus lateraler Richtung oder aus 50° relativ zur Mediansagitalebene. Andere Beispiele für Art-Informationen sind eine abzuklärende Diagnosehypothese. So kann beispielsweise die Eingabe "HWS-Trauma" automatisch zur Folge haben, dass eine Datenbank abgefragt wird, die zum Abklären dieser spezifischen Diagnosehypothese bestimmte Abbildungsbedingungen vorsieht. Beispielsweise kann bei der Eingabe von "HWS-Trauma" eine Abbildung von C1-C7 sowohl von vorne als auch lateral vorgesehen sein, um eine Kyphose der HWS beurteilen zu können. Vorzugsweise wird also mittels der Art-Information durch Eingabe bestimmter Begriffe automatisch ein spezifischer Teil der anatomischen Struktur ausgewählt und automatisch bestimmte Abbildungsbedingungen und insbesondere eine Mindestanzahl von Abbildungen festgelegt. Beispielsweise können bei Verdacht auf Osteoporose im Bereich L4/L5 andere Abbildungsbedingungen gewünscht sein als beim Verdacht eines Bandscheibenvorfalls im Bereich L4/L5. Bei einer Änderung der Abbildungsbedingungen von Abbildung zu Abbildung wird also vorzugsweise ebenfalls auf die vorgenannten Art-Informationen zurückgegriffen.

Sollte nach einer Änderung der Abbildungsbedingungen, die erfindungsgemäß insbesondere automatisch erfolgt, mit der nächsten Abbildung nicht das spezifische Teil (in gewünschter Art und Weise) erfasst worden sein, so werden vorzugsweise bereits zuvor erwähnte Schritte wiederholt. Insbesondere werden aus der zweiten Abbildung oder den weiteren Abbildungen ebenfalls die dabei jeweils geltenden Abbildungsbedingungen insbesondere mittels der Datenbank bestimmt. Basierend auf den neu bestimmten Abbildungsbedingungen werden dann die neuen Abbildungsbedingungen für die nächste Abbildung so bestimmt, dass das spezifische Teil (möglichst in der gewünschten Art und Weise) möglichst erfasst wird.

Bei Abgleich des abgebildeten Teils mit den Daten der Datenbank wird vorzugsweise so vorgegangen, dass angenommen wird, dass das abgebildete Teil sich in der Nähe des spezifischen Teils befindet. Auf diese Art und Weise kann die Datenverarbeitung und der Suchvorgang beschleunigt werden.

Es handelt sich bei den durchgeführten Abbildungen um zweidimensionale Abbildungen, wobei durch eine Abfolge von zweidimensionalen Abbildungen ein dreidimensionales Modell des spezifischen Teils erstellt wird. Erfindungsgemäß soll die Abfolge dieser zweidimensionalen Abbildungen optimiert werden, um das dreidimensionale Modell mit möglichst wenigen Abbildungen und in gewünschter Qualität zu erstellen. Durch Minimierung der Zahl der Abbildungen kann zum Beispiel bei Röntgenabbildungen die Belastung des Patienten verringert werden.

Weiter umfasst die Datenbank vorzugsweise für verschiedene gespeicherte Teile verschiedene jeweils zur Abbildung des gespeicherten Teils geeignete Abbildungsbedingungen. Insbesondere ist vorzugsweise die Datenbank so gestaltet, dass bei einer gewünschten dreidimensionalen Modellierung eines der gespeicherten Teile bestimmte bevorzugte Abbildungsbedingungen verwendet werden, um ein dreidimensionales Modell desjenigen Teils der anatomischen Struktur zu erstellen, der dem gespeicherten Teil entspricht. Vorzugsweise wird basierend auf dem bekannten spezifischen Teil die Datenbank abgefragt und mit einem gespeicherten Teil abgeglichen. Für dieses gespeicherte Teil werden dann die geeigneten Abbildungsbedingungen aus der Datenbank abgefragt und die Abbildungen werden entsprechend diesen gespeicherten Abbildungsbedingungen vorgenommen. Insbesondere werden von Abbildung zu Abbildung die Bedingungen so geändert, dass sie möglichst den gespeicherten Abbildungsbedingungen entsprechen, insbesondere der Abfolge der gespeicherten Abbildungsbedingungen.

Im Falle einer dreidimensionalen Modellierung eines spezifischen Teils wird alternativ oder zusätzlich eine Änderung der Abbildungsbedingungen und eine erneute Abbildung dann vorgenommen, falls mit den bisher erzielten Abbildungen kein dreidimensionales Modell des spezifischen Teils erzielt werden kann oder nicht in der gewünschten Qualität erzielt werden kann. Eine erneute Abbildung kann beispielsweise an die Bedingung geknüpft werden, dass die Berechnung eines dreidimensionalen Modells des spezifischen Teils nicht konvergiert oder vorbestimmte Kriterien, insbesondere eine ausreichend hohe Genauigkeit oder ausreichend geringe Fehlerbehaftung des dreidimensionalen Modells nicht erfüllt. Die erneute Abbildung wird dabei natürlich vorzugsweise so gestaltet, dass sich die Abbildungsbedingungen von denjenigen der vorherigen Abbildung unterscheiden. Insbesondere wird die Abbildungsrichtung geändert. Beispielsweise kann die Abbildungsrichtung so geändert werden, dass sie möglichst weit von allen bisherigen Abbildungsrichtungen abweicht, um so zusätzliche Information für die Modellierung des dreidimensionalen Modells zu erzielen. Auch kann eine spezifische Sequenz von Abbildungsbedingungen, insbesondere Abbildungsrichtungen vorgegeben werden, die dann beendet wird, wenn ein 3-D-Modell gewünschter Qualität mit den gegebenen Abbildungen erzielt werden kann.

Vorzugsweise wird ein Lernalgorithmus verwendet, um die Änderung der Abbildungsbedingungen zu optimieren und um insbesondere aus vorangegangenen Abbildungen zur Modellierung eines früheren spezifischen Teils zu lernen. Insbesondere hierzu werden vorzugsweise die zur Abbildung eines spezifischen Teils verwendeten Abbildungsbedingungen gespeichert. Insbesondere wird zusätzlich die Anzahl der Abbildungen gespeichert, die notwendig waren, um das 3-D-Modell mit gewünschter Genauigkeit zu erzielen. Gemäß dem Lernalgorithmus können beispielsweise diejenigen Abbildungsbedingungen für zukünftige Abbildungen ausgewählt werden, die mit der geringsten Zahl von Abbildungen zu dem gewünschten Ergebnis geführt haben.

Gemäß einer weiteren Ausführungsform werden bei der zuvor erwähnten Sequenz von Abbildungen für ein spezifisches Teil und/oder gespeichertes Teil, um ein 3-D-Modell zu erzielen, die Abbildungsbedingungen von Abbildungssequenz zu Abbildungssequenz variiert, um im Rahmen des zuvor erwähnten Prozesses die Abbildungssequenzen zu optimieren. Dabei wird dann auf diejenige Abbildungssequenz zurückgegriffen, die die beste Qualität und/oder die geringste Anzahl von Abbildungen bei der Erstellung eines 3-D-Modells erzielt hat.

Vorzugsweise wird bei der Bestimmung der Abbildungsbedingungen alternativ oder zusätzlich auf Information zurückgegriffen, aus der sich auf die Lage und/oder Orientierung des spezifischen Teils relativ zu dem Aufnahmegerät schließen lässt. Beispielsweise kann eine Markereinrichtung am Patienten, insbesondere an der anatomischen Struktur angebracht sein und/oder mit dem Patienten verbunden sein. Eine Markereinrichtung besteht insbesondere aus Markerelementen, wie zum Beispiel Markerkugeln, die eine vorbestimmte räumliche Beziehung zueinander haben und Signale reflektieren oder aussenden. Die von den Markern ausgehenden Signale werden durch eine Detektionseinrichtung, die beispielsweise Teil eines Navigationssystems (zum Beispiel IGS) sein kann, detektiert. Eine Datenverarbeitungseinrichtung kann basierend auf den detektierten Signalen dann die Lage der Markereinrichtung zum Beispiel relativ zur Detektionseinrichtung und/oder in einem bestimmten Bezugssystem (zum Beispiel Raum, in dem die Untersuchung stattfindet) bestimmen. Ist die Markereinrichtung relativ zu einem Gerät oder zu einer anatomischen Struktur oder einem Patienten registriert, so kann somit auch die Lage des Patienten oder der anatomischen Struktur bestimmt werden. Auch können die Markereinrichtungen an einer Ausrichthilfe oder an einer Liege befestigt sein, auf der der Patient liegt oder bezüglich der der Patient ausgerichtet wird. Durch Detektion der Markereinrichtung und unter Annahme einer üblichen Lage des Patienten kann somit auf die Lage des Patienten relativ zu dem Abbildungsgerät geschlossen werden. Auch kann am Abbildungsgerät selber eine Markereinrichtung angebracht werden, um so die relative Lage und Ausrichtung des Abbildungsgeräts relativ zum Patienten, insbesondere den spezifischen Teil der anatomischen Struktur zu schließen. Somit kann mittels Detektion der Markereinrichtung und Auswertung der detektierten Signale, insbesondere mittels eines Navigationssystems auf die Abbildungsbedingungen durch Detektion der Markereinrichtung und bei bekannter relativer Lage zwischen Abbildungsgerät und Markereinrichtung geschlossen werden. Die so bestimmten Abbildungsbedingungen können erfindungsgemäß verwendet werden, ohne dass auf die sich aus dem abgebildeten Teil ergebenden Informationen zur Bestimmung der Abbildungsbedingungen zurückgegriffen wird. Sie können aber auch als Ausgangspunkt und/oder zusätzliche Information zur Bestimmung der Abbildungsbedingungen basierend auf dem abgebildeten Teil verwendet werden, um somit schneller und effektiver die Abbildungsbedingungen (unter denen das abgebildete Teil erfasst wurde) bestimmen zu können. Auch können die mittels der Markereinrichtungen bestimmten Abbildungsbedingungen und die basierend auf der Abbildung und der Datenbank bestimmten Abbildungsbedingungen miteinander verglichen werden und bei Abweichungen voneinander beispielsweise über ein gewichtetes Verfahren Abbildungsbedingungen als gültig bestimmt werden, die dann als Ausgangspunkt für weitere Abbildungen und Änderungen der Abbildungsbedingungen verwendet werden.

Die vorliegende Erfindung bezieht sich nicht nur auf die oben genannten Verfahren, sondern auch auf ein Programm, das einen Computer veranlasst, das oder die Verfahren umzusetzen, wenn es auf dem Computer abläuft. Ebenso ist die Erfindung auf entsprechende Computerprogrammspeichermedien, die das Programm speichern oder auf eine Signalwelle, die das Programm überträgt (beispielsweise eine Übertragung des Programms über das Internet, wie zum Beispiel Download) gerichtet.

Die Erfindung umfasst ebenfalls eine Vorrichtung, die ausgebildet ist, das erfindungsgemäße Verfahren auszuführen. Die Vorrichtung umfasst das Abbildungsgerät, wie beispielsweise ein Röntgengerät oder ein Ultraschallgerät. Das Abbildungsgerät ist ausgebildet, einen Teil einer anatomischen Struktur abzubilden, also beispielsweise mit einem Sensor zu erfassen. Die Abbildungen sind zweidimensional. Die Abbildung erfolgt unter bestimmten Abbildungsbedingungen, wie bereits oben ausgeführt wurde.

Weiter umfasst die Vorrichtung die Datenbank, in der Informationen über die anatomische Struktur und insbesondere Teile der anatomischen Struktur, ihre Beschaffenheit und insbesondere ihre anatomischen Details gespeichert sind. Weiter umfasst die Vorrichtung eine Datenverarbeitungseinrichtung, die insbesondere Bestimmungsschritte und/oder Berechnungsschritte durchführt. Die Datenverarbeitungseinrichtung ist ausgebildet, die Datenbank basierend auf der mit dem Abbildungsgerät durchgeführten Abbildung, also basierend auf dem erfassten abgebildeten Teil, abzufragen. Die Daten über die Abbildung werden so vorzugsweise von dem Abbildungsgerät zu der Datenverarbeitungseinrichtung übermittelt. Die Datenverarbeitungseinrichtung bestimmt weiter mittels der in der Datenbank gespeicherten Informationen zumindest einen Teil der Abbildungsbedingungen.

Die erfindungsgemäße Vorrichtung umfasst weiter vorzugsweise eine Eingabeeinrichtung, zum Beispiel Tastatur, Wechselspeicher, Nutzerschnittstelle, die es insbesondere erlaubt, Informationen über das spezifische Teil einzugeben, insbesondere Bestimmungs-Informationen, die es erlauben, das spezifische Teil in der Datenbank zu identifizieren und/oder zu bestimmen. Die Datenverarbeitungseinrichtung bestimmt weiter vorzugsweise basierend auf den bestimmten Abbildungsbedingungen eine durchzuführende Änderung der Abbildungsbedingungen, um mit der nächsten Abbildung das spezifische Teil geeignet zu erfassen.

Die Erzeugung eines dreidimensionalen Modells einer anatomischen Struktur ist aus dem Stand der Technik bekannt. Hierbei wird auf die EP 1 611 863 (US 2006004284) der Anmelderin hingewiesen . Dort wird ein Verfahren zum Erzeugen eines dreidimensionalen Modells einer anatomischen Struktur mit Hilfe eines chirurgischen Navigationssystems offenbart. Dabei werden zwei Fluoroskopiebilder verwendet. Bei diesem bekannten Verfahren zum Erzeugen eines dreidimensionalen Modells einer anatomischen Struktur wird die Position und Orientierung des dreidimensionalen Modells relativ zu einer Markereinrichtung bestimmt. Die Position der Markereinrichtung wird mittels eines chirurgischen Navigationssystems bestimmt und verfolgt. Das Verfahren umfasst folgende Schritte:
Eine Markereinrichtung, die von einer Detektionseinrichtung, insbesondere einem Navigationssystem detektiert werden kann, wird an einer anatomischen Struktur angebracht.
   - Die Position der Landmarken der anatomischen Struktur wird mittels eines sogenannten Pointers (Zeigegerät mit Markereinrichtungen, die es erlauben, die Spitze des Pointers zu lokalisieren) identifiziert;
   - ein grobes dreidimensionales Modell der anatomischen Struktur und ihrer Position im Raum wird berechnet;
   - ein C-Bogen, der von dem Navigationssystem detektiert wird und dessen Position von dem Navigationssystem bestimmt wird, wird manuell in wenigstens zwei Positionen bewegt. Die zwei Positionen sind geeignet, um Röntgenbilder der anatomischen Struktur aufzunehmen. Die Position des C-Bogens, und insbesondere des Abbildungsteils des C-Bogens relativ zu der anatomischen Struktur wird von dem Navigationssystem angezeigt, um einen Benutzer zu führen;
   - ein verbessertes 3-D-Modell der anatomischen Struktur und seine räumliche Beziehung zu einer Markereinrichtung wird berechnet.

Das erfindungsgemäße Verfahren kann einzelne oder alle Schritte des oben genannten Verfahrens umfassen. Insbesondere können mittels eines Pointers Landmarken der anatomischen Struktur, insbesondere des spezifischen Teils abgegriffen werden, um mittels einer Detektionseinrichtung, insbesondere eines Navigationssystems die Position der anatomischen Struktur, insbesondere des spezifischen Teils relativ zu dem Abbildungsgerät zu bestimmen, um so gegebene Abbildungsbedingungen zu bestimmen.

Eine bewegliche Steuerung einer Röntgenquelle und eines Röntgenziels ist aus US6200024B1 bekannt. Eine derartige Steuerbarkeit der Bewegung des Abbildungsgeräts soll auch gemäß einer Ausführungsform der vorliegenden Erfindung umfasst sein. Ist für die Abbildung eine Quelle und ein Ziel erforderlich, soll insbesondere eine spezifische räumliche Beziehung zwischen Quelle und Ziel bevorzugt aufrechterhalten werden, wenn eines der beiden verfahren wird, um die Abbildungsbedingungen zu ändern.

Allgemein wird in EP 1 056 572 B1 ein Verfahren zum Registrieren der Position eines Roboters relativ zu einem Werkstück offenbart, wobei der Roboter eine Abbildungseinheit trägt. Die beliebige Steuerung eines Abbildungsgeräts, das an einem Roboterarm befestigt sein kann, soll ebenfalls erfindungsgemäß umfasst sein.

Bei der folgenden Beschreibung von Ausführungsformen werden weitere Merkmale und Vorteile der Erfindung offenbart.
Figur 1 zeigt schematisch den Aufbau einer erfindungsgemäßen Vorrichtung.
Figur 2 zeigt einen Ablauf eines erfindungsgemäßen Verfahrens.
Figur 3 zeigt schematisch das Prinzip der Änderung der Abbildungsbedingungen.

Vorteilhaft erlaubt das erfindungsgemäße Verfahren ein spezifisches Teil einer anatomischen Struktur zu erfassen, insbesondere ein 3-D-Modell des spezifischen Teils. Vorzugsweise wird das 3-D-Modell mittels des erfindungsgemäßen Verfahrens registriert, insbesondere die Position des 3-D-Modells in einem Bezugssystem mittels eines Navigationssystems erfasst.

Insbesondere zusätzlich zu einem oder mehreren Schritten des oben in Zusammenhang mit EP 1 611 863 beschriebenen Verfahrens werden einer oder mehrere der folgenden Schritte durchgeführt:
- Basierend auf einem groben dreidimensionalen Modell der anatomischen Struktur, insbesondere des spezifischen Teils und vorzugsweise auch basierend auf (mittels einer Detektionseinrichtung und/oder eines Navigationssystems erfassten) Navigationsdaten werden optimale Positionen des Abbildungsgeräts, beispielsweise des Röntgengeräts in einem C-Bogen berechnet. Navigationsdaten sind dabei insbesondere Daten, die mittels Markereinrichtungen und/oder Pointer erfasst wurden, die mit der anatomischen Struktur in Zusammenhang stehen, also insbesondere an dieser angebracht sind oder diese abtasten.
- Vorzugsweise werden unterschiedliche Kriterien für die räumliche Beziehung unter den Objekten, insbesondere räumliche Beziehung des Abbildungsgeräts relativ zu dem spezifischen Teil genutzt, um die optimale Position der Abbildungsvorrichtung zu bestimmen. Beispielsweise werden für verschiedene Teile der anatomischen Struktur andere Abbildungsrichtungen vorgeschlagen, um angepasst an den jeweiligen Teil der anatomischen Struktur die bestmöglichen dreidimensionalen Modelle zu erzielen. Wie bereits oben ausgeführt, werden bevorzugt die jeweiligen Abbildungsrichtungen in einer Datenbank abgespeichert.
- Vorzugsweise ist das Abbildungsgerät so gestaltet, dass die Position einer Abbildungseinheit des Abbildungsgeräts, die die eigentliche Abbildung durchführt, verstellbar ist. Vorzugsweise umfasst das Abbildungsgerät hierzu einen steuerbaren Roboterarm oder eine steuerbare und gelenkige Mechanik, die es erlaubt, die Abbildungseinheit in neue Positionen und/oder Orientierung relativ zu dem spezifischen Teil zu bringen, um so neue Abbildungsbedingungen einzustellen.
- Falls eine Berechnung des dreidimensionalen Modells und eine Bestimmung der räumlichen Position und Orientierung fehlschlägt, werden vorzugsweise neue Positionen berechnet, die die Abbildungseinheit einnehmen soll.
- Vorzugsweise werden Positionen und/oder räumliche Beziehungen von Objekten und/oder das Ergebnis einer Registrierung gespeichert, das heißt, ob die Registrierung fehlgeschlagen ist oder erfolgreich war. Vorzugsweise enthält das erfindungsgemäße System oder die erfindungsgemäße Vorrichtung eine Datenbank, die bei zukünftigen Berechnungen zum Berechnen optimaler Abbildungsbedingungen (beispielsweise Position und Orientierung der Abbildungseinheit) verwendet werden kann.
- Vorteilhaft wird erfindungsgemäß eine automatische Positionierung der Abbildungseinheit durchgeführt. Auf diese Art und Weise kann Zeit gegenüber einer manuellen Einstellung gespart werden. Außerdem kann aufgrund der erfindungsgemäßen Berechnung der optimalen Abbildungsbedingungen die Anzahl der notwendigen Aufnahmen reduziert werden. Vorzugsweise werden Positionen weiterer Geräte erfasst und/oder abgespeichert. Die Erfassung der weiteren Geräte, die insbesondere eine Abbildung stören können, erfolgt beispielsweise ebenfalls über Markereinrichtungen, die an den Geräten angebracht sind, und von einem erfindungsgemäßen Navigationssystem erfasst werden. Ist die Position der Geräte abgespeichert, so wird die abgespeicherte Position vorzugsweise durch das erfindungsgemäße Verfahren genutzt oder durch die erfindungsgemäße Vorrichtung abgefragt, um so zu berechnen, ob eines der Geräte die Abbildung stört, das heißt insbesondere in einem räumlichen Bereich liegt, der von der Abbildungseinheit erfasst wird. Vorzugsweise werden die Abbildungsbedingungen so berechnet, dass der Abbildungsvorgang nicht durch weitere, insbesondere zwischen der Abbildungseinheit und dem spezifischen Teil liegende Elemente oder Geräte gestört wird.
- Bei einer bevorzugten Ausführungsform sind die Röntgenquelle und das Röntgenziel, also das röntgenstrahlungsempfindliche Gerät an getrennten beweglichen Armen montiert. Auf diese Art und Weise ist eine im Vergleich zum C-Bogen flexiblere Auswahl der Beobachtungsrichtungen und somit der Abbildungsbedingungen möglich. Insbesondere können verschiedenste Abbildungsrichtungen bei einer höheren Anzahl von Freiheitsgraden der Bewegung der Betätigungsarme erzielt werden.
- Vorzugsweise gibt es einen direkten Datenaustausch zwischen dem Abbildungsgerät und der Datenverarbeitungseinrichtung. Falls die bisher erfassten Abbildungen oder die bisher erfasste Abbildung nicht die Erfordernisse eines Bildanalysierungsalgorithmuses erfüllt, werden alternative Abbildungsbedingungen berechnet und die betätigbaren Arme des Abbildungsgeräts werden automatisch gesteuert, bis der korrekte Bildinhalt erzielt worden ist. Dies gilt beispielsweise, falls mit der ersten Abbildung ein falscher Ausschnitt der anatomischen Struktur erzielt wurde. Die Abbildungsbedingungen werden dann so geändert, dass mit der nächsten Abbildung der korrekte Ausschnitt erzielt wird.
- Vorzugsweise wird das bereits berechnete dreidimensionale Modell genutzt, um Abbildungsbedingungen für die nächste Abbildung zu berechnen, um so mit einer weiteren Abbildung das zweidimensionale Modell zu optimieren. Das dreidimensionale Modell ist beispielsweise ursprünglich nur ein grobes Modell basierend auf Datenbankinformationen, dessen räumliche Lage insbesondere nur aufgrund einer groben Vorregistrierung (beispielsweise mittels Pointer und Landmarken) erfolgt ist. Vorzugsweise kann durch das erfindungsgemäße Verfahren eine genaue Registrierung des dreidimensionalen Modells relativ zu dem Abbildungsgerät und/oder in einem Bezugssystem (zum Beispiel des Navigationssystems) erfolgen.
- Vorteilhaft kann durch die Erfindung eine automatische Positionierung der Abbildungseinheit erfolgen. Dadurch wird es einem Bediener ermöglicht, sich von einem gesundheitsgefährdenden Abbildungsgerät, wie beispielsweise einem Röntgengerät entfernt zu halten, um so seine Belastung zu reduzieren.
- Vorteilhaft kann durch die automatisierte Änderung der Abbildungsbedingungen die Chance für das Konvergieren der Berechnung eines 3-D-Modells erhöht werden.
- Schließlich kann durch das Abspeichern von Abbildungsbedingungen, insbesondere Positionsdaten der Abbildungseinheit von früheren Abbildungsvorgängen, und von Abbildungssequenzen Erfahrung gesammelt werden, um die Berechnung zukünftiger Abbildungsbedingungen zu optimieren.

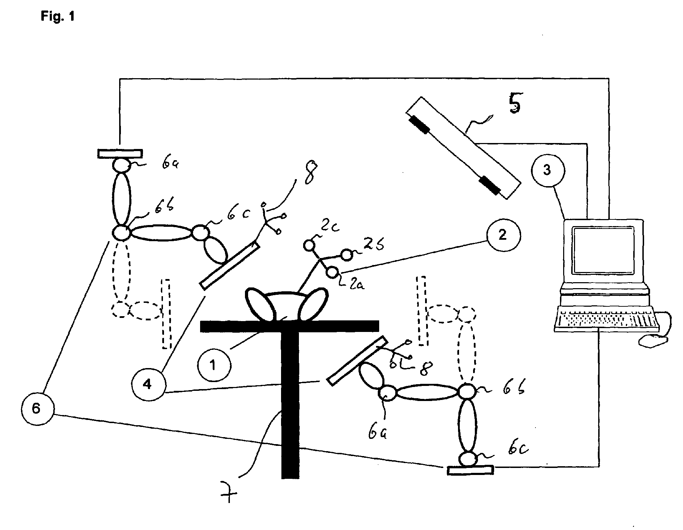

Die Figur 1 zeigt beispielhaft eine erfindungsgemäße Vorrichtung und erlaubt das erfindungsgemäße Verfahren beispielhaft zu erläutern. Ein mögliches Ziel der Erfindung ist es ein dreidimensionales Modell eines spezifischen Teils 1 einer anatomischen Struktur, die auf einem T-förmigen Tisch 7 liegt, zu erzeugen und insbesondere die Position und/oder die Orientierung des spezifischen Teils 1 zu bestimmen. Diese Position und/oder Orientierung kann insbesondere in einem Bezugssystem bestimmt werden, in dem das spezifische Teil ruht oder in dem eine Detektionseinrichtung 5 der erfindungsgemäßen Vorrichtung ruht. Insbesondere kann die Position und/oder Orientierung des spezifischen Teils 1 relativ zu einer Markereinrichtung 2 (mit Markerkugeln 2a, 2b, 2c) bestimmt werden, deren Position durch die Detektionseinrichtung 5 detektiert wird und durch die Datenverarbeitungseinrichtung 3 berechnet wird. Die Detektionseinheit 5 und die Datenverarbeitungseinrichtung 3 können zusammen ein Navigationssystem 3, 5 bilden, das zum Navigieren beispielsweise von chirurgischen Instrumenten verwendet werden kann. Die erfindungsgemäße Vorrichtung umfasst beispielsweise das vorgenannte Navigationssystem. Die erfindungsgemäße Vorrichtung umfasst vorzugsweise weiter Abbildungsgeräte 6, die Abbildungseinheiten 4 umfassen, deren Position mittels Gelenke 6a, 6b, 6c veränderbar ist. Eine beispielhafte alternative Position der Abbildungseinheiten 4 ist gestrichelt eingezeichnet. Die Abbildungsgeräte 6 liefern zweidimensionale Abbildungen zu der Datenverarbeitungseinrichtung 3. Abgebildet wird die anatomische Struktur und möglichst das spezifische Teil 1. Das Abbildungsgerät 6 umfasst vorzugsweise, wie schematisch eingezeichnet ist, einen beweglichen Arm mit Gelenken 6a, 6b, 6c, der die Abbildungseinheit 4 trägt, um so die Abbildungsbedingungen einzustellen. Die Position der Abbildungseinheit und somit der Arm des Abbildungsgeräts 6 wird vorzugsweise ebenfalls mittels der Datenverarbeitungseinrichtung 3 gesteuert.

Vorzugsweise werden die Abbildungsbedingungen, also insbesondere die Position und/oder die Ausrichtung der Abbildungseinheit 4 durch die Datenverarbeitungseinheit 3 berechnet, um so die Abbildungsbedingungen für weitere Abbildungen zu bestimmen, insbesondere eine Sequenz von Abbildungsbedingungen zu bestimmen. Die Datenverarbeitungseinrichtung enthält hierzu die anatomische Datenbank, die es erlaubt, das abgebildete Teil zu identifizieren und so die Abbildungsbedingungen zu bestimmen. Die Sequenz von Abbildungsbedingungen wird vorzugsweise so optimiert, um die Berechnung eines dreidimensionalen Modells des spezifischen Teils 1 der anatomischen Struktur mit möglichst wenigen Abbildungen zu ermöglichen oder zu optimieren. Insbesondere soll die Lage des dreidimensionalen Modells des spezifischen Teils in einem Bezugssystem des Navigationssystems 3, 5, also beispielsweise relativ zu der Markereinrichtung 2 bestimmt werden. Hierzu wird die Markereinrichtung 2 beispielsweise so gestaltet, dass sie sowohl von der Detektionseinrichtung 5 detektiert werden als auch durch das Abbildungsgerät 6 abgebildet werden kann. Beispielsweise können die Markerelemente, insbesondere Markerkugeln 2a, 2b, 2c sowohl optisch detektierbar sein, also beispielsweise Infrarotlicht passiv reflektieren als auch für Röntgenstrahlen undurchlässig sein, um somit von einem Röntgengerät, das als Beispiel für ein Abbildungsgerät dient, ebenfalls detektierbar zu sein. Somit kann die relative Lage der Markereinrichtung 2 relativ zur anatomischen Struktur mittels des Abbildungsgeräts 6 erfasst werden. Da außerdem die Lage der Markereinrichtung 2 durch das Navigationssystem 3, 5 in einem beliebigen Bezugssystem (beispielsweise Operationsraum) erfasst wird, kann somit die Lage des spezifischen Teils 1 in dem Bezugssystem, beispielsweise Operationssaal durch die erfindungsgemäße Vorrichtung bestimmt werden. Die Position der Abbildungseinheiten 4 kann durch die Steuerung der Position zum Beispiel mittels der Datenverarbeitungseinrichtung bekannt sein. Um die Position der Abbildungseinheiten 4 überprüfen und/oder erfassen zu können, können auch an diesen Abbildungs-Markereinrichtungen 8 angebracht sein, die ebenfalls von der Detektionseinrichtung 5 erfasst werden, um die relative Lage zwischen Abbildungseinheit 4 und der Markereinrichtung 2 bestimmen zu können. Auch kann so Information über die gegebene Abbildungsbedingung gesammelt werden, die umso genauer ist, je genauer die Lage zwischen der Markereinrichtung 2 und dem spezifischen Teil 1 bekannt ist. Diese Information kann dann zur Bestimmung der Abbildungsbedingungen verwendet werden oder in die Bestimmung der Abbildungsbedingungen mittels der Datenbank und basierend auf dem abgebildeten Teil mit einfließen.

Vorzugsweise kann ein dreidimensionales Modell eines spezifischen Teils 1 einer anatomischen Struktur erzeugt werden und die Position des spezifischen Teils und seine Orientierung relativ zu wenigstens einer Markereinrichtung 2 bestimmt werden, wobei die Position der Markereinrichtung durch das chirurgische Navigationssystem 3, 5 verfolgt werden kann. Insbesondere kann die Position des Abbildungsgeräts 6 genauer der Abbildungseinheit 4 durch die Datenverarbeitungseinrichtung 3 und insbesondere durch das Navigationssystem 3, 5 zumindest teilweise gesteuert werden. Insbesondere können so die Positionen der Abbildungseinheiten optimiert werden, um geeignete Abbildungsbedingungen anzufahren, die zur Berechnung eines dreidimensionalen Modells des spezifischen Teils geeignet sind. Insbesondere kann die Abbildungseinheit geeignet in ihrer Orientierung und Position relativ zur Markereinrichtung 2 positioniert werden.

Gewünschte Positionen und/oder Orientierungen der Abbildungseinheit können beispielsweise durch die räumliche Beziehung zwischen Objekten beschrieben werden, wobei die Lage der Objekte zum Beispiel durch die Detektionseinrichtung 5 oder das Navigationssystem 3, 5 erfasst werden kann und wie folgt verwendet werden kann:
1. Die räumliche Beziehung kann beispielsweise durch einen Winkel zwischen der Abbildungseinheit und beispielsweise der Längsachse des Patientenkörpers beschrieben werden.
2. Die räumliche Beziehung kann durch einen Winkel zwischen der Abbildungseinheit 4 und einer Symmetrieebene der anatomischen Struktur, beispielsweise einer Symmetrieebene des Beckens beschrieben werden.
3. Die räumliche Beziehung kann durch einen Abstand zwischen der Abbildungseinheit und dem spezifischen Teil 1 der anatomischen Struktur beschrieben werden. Dieser Abstand kann im Hinblick auf die gegebenen Abbildungseigenschaften eines Abbildungsgeräts insbesondere so gewählt werden, dass das spezifische Teil eine bestimmte Größe in der Abbildung einnimmt.
4. Die räumliche Beziehung kann durch einen relativen Winkel oder eine relative Position zwischen der Abbildungseinheit und dem Operationstisch beschrieben werden.
5. Die räumliche Beziehung kann auch durch Angabe einer relativen Position zwischen der Abbildungseinheit relativ zu anderen Geräten (zum Beispiel im Operationsraum) angegeben werden, deren Position von dem Navigationssystem oder einer Detektionseinrichtung 5 oder irgendeinem anderen Detektionsgerät, das auch in der Abbildungseinheit integriert sein kann, detektiert und erfasst wird.
6. Räumliche Beziehung zwischen der Abbildungseinheit und dem spezifischen Teil kann auch basierend auf einem maximalen Winkel beschrieben werden, der zwischen Abbildungsprojektionen gegeben ist, die bei einer aktuellen Position oder Sollposition vorhanden sind, und Abbildungsprojektionen, die sich bei einer vorhergehenden Abbildung ergeben haben.

Ein Verfahren zum automatischen Positionieren einer Abbildungseinheit oder des Abbildungsgeräts umfasst insbesondere die folgenden Schritte:
- Ein erster Satz von wenigstens zwei Positionen der Abbildungseinheit wird wenigstens teilweise basierend auf Anfangsinformation über die anatomische Struktur und basierend auf der Position und Orientierung der anatomischen Struktur, die mittels des Navigationssystems erfasst wurde, bestimmt.
- Die Abbildungseinheit wird mittels einer Steuerung bewegt, die beispielsweise die Haltestruktur (Gelenke) der Abbildungseinheit steuert. Die Steuerung der Bewegung erfolgt vorzugsweise basierend auf Daten des Navigationssystems, um die Abbildungseinheit in die als geeignet bestimmten Positionen zu bringen und ein Bild an jeder Position zu erfassen.
- Vorzugsweise werden wenigstens zwei Bilder als Ausgangspunkt zur Berechnung eines dreidimensionalen Modells des spezifischen Teils der anatomischen Struktur verwendet.
- Das 3-D-Modell wird berechnet und die Position und Orientierung des 3-D-Modells relativ zu einer Markereinrichtung, die an dem spezifischen Teil und/oder der anatomischen Struktur angebracht ist, wird vorzugsweise ebenfalls berechnet.
- Vorzugsweise wird ein registriertes 3-D-Modell zur Navigation, insbesondere bei chirurgischen Eingriffen, verwendet.

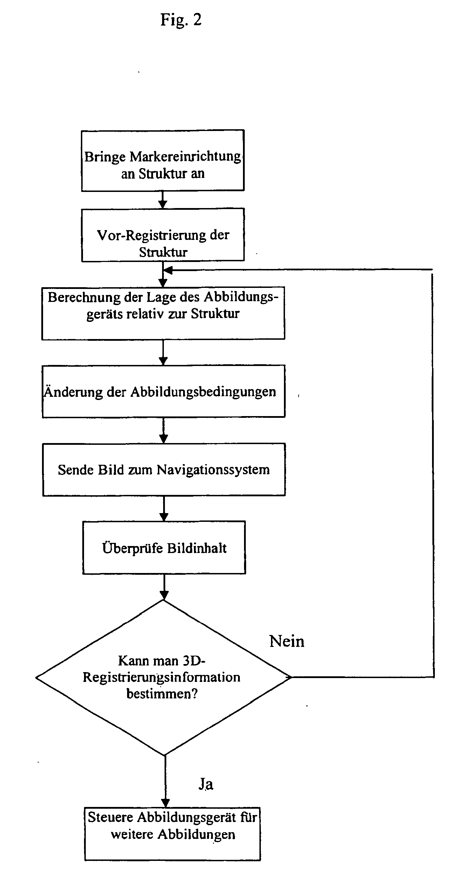

Figur 2 zeigt schematisch einen Ablauf zur Bestimmung eines 3-D-Modells. Zuerst wird vorzugsweise eine Markereinrichtung an der anatomischen Struktur bzw. dem Patienten angebracht. Danach erfolgt eine Vorregistrierung, also insbesondere eine Bestimmung der Lage und Orientierung der anatomischen Struktur, insbesondere des spezifischen Teils im Raum. Dann erfolgt eine Berechnung der relativen Lage und/oder Orientierung der Abbildungseinheit relativ zur anatomischen Struktur, insbesondere relativ zum spezifischen Teil. Anschließend wird das Abbildungsgerät, insbesondere die Abbildungseinheit betätigt oder erneut betätigt, um eine Abbildung mit einer bestimmten Projektion zu erfassen, also unter bestimmten Abbildungsbedingungen zu erfassen. Anschließend wird das erfasste Bild zum Navigationssystem gesendet. Dort wird der Bildinhalt überprüft, insbesondere ob er vorgegebene Bedingungen erfüllt, also insbesondere ob er das spezifische Teil wiedergibt. Im nächsten Schritt wird dann überprüft, ob es die zur Verfügung stehenden Abbildungen erlauben, ein dreidimensionales Modell zu berechnen und insbesondere das spezifische Teil dreidimensional in einem Bezugssystem des Navigationssystems zu registrieren. Ist dies nicht der Fall, so werden erneut die obigen Schritte ab der Berechnung der Lage des Abbildungsgeräts relativ zur Struktur, insbesondere relativ zum spezifischen Teil wiederholt, bis ein dreidimensionales Modell berechnet werden kann und/oder eine dreidimensionale Registrierungsinformation bestimmt werden kann. Ist diese Bedingung erfüllt, so erfolgt diese Berechnung. Insbesondere kann das Abbildungsgerät angesteuert werden, um weitere Abbildungen unter anderen Abbildungsbedingungen durchzuführen.

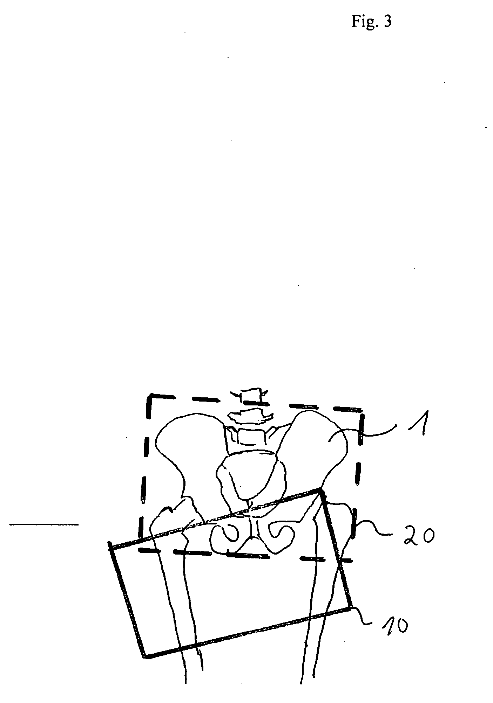

Fig. 3 zeigt mit einer ersten Abbildung 10 und einer zweiten Abbildung 20 erfasste Teile einer anatomischen Struktur. Das mit der Abbildung zu erfassende spezifische Teil ist das Becken, das mit der zweiten Abbildung 20 erfasst wird. Die erste Abbildung 10 erfasst nur einen Teil des Beckens. Der mit der ersten Abbildung 10 abgebildete Teil, der Teile der Oberschenkel enthält, wird mit einer Datenbank abgeglichen, um zu erfassen, welcher Teil der anatomischen Struktur mit der Abbildung 10 erfasst wurde und wie dieser relativ zum spezifischen Teil liegt. Basierend hierauf wird dann die Abbildungsbedingung geändert, also insbesondere die Position einer Abbildungseinheit 4 verfahren, so dass mit einer zweiten Abbildung 20 das spezifische Teil, das heißt in dem Beispiel das Becken, erfasst wird. Somit kann basierend auf der Auswertung der mit der ersten Abbildung erfassten anatomischen Information die Abbildungsbedingung so geändert werden, dass mit einer zweiten Abbildung das spezifische Teil in gewünschter Weise erfasst wird.

## Patentansprüche

1. Verfahren zum Erfassen eines spezifischen Teils (1) einer anatomischen Struktur mittels einer Abfolge von Abbildungen,
mit folgenden Schritten:
a) eine Abbildung (10) eines abgebildeten Teils der anatomischen Struktur wird unter Abbildungsbedingungen erstellt;
b) eine Datenbank (3), in der Informationen über die anatomische Struktur abgespeichert sind, wird basierend auf der Abbildung (10) abgefragt, um mittels der gespeicherten Informationen zumindest einen Teil der Abbildungsbedingungen zu bestimmen;
**dadurch gekennzeichnet, dass**
die Abbildungen zweidimensionale Abbildungen sind und mittels der Abfolge von Abbildungen ein dreidimensionales Modell des spezifischen Teils erstellt wird, wobei die Datenbank (3) für verschiedene gespeicherte Teile der anatomischen Struktur verschiedene geeignete Abbildungsbedingungen umfasst, die zur Erstellung eines dreidimensionalen Modells basierend auf den unter den geeigneten Abbildungsbedingungen erstellten zweidimensionalen Abbildungen geeignet sind, und
mit Hilfe der Datenbank (3) bestimmt wird, welche von den gespeicherten Teilen zu dem spezifischen Teil (1) passt, um die geeignete Abbildungsbedingung für eine erste und/oder nächste Abbildung basierend auf dem als passend bestimmten gespeicherten Teil und den diesen zugeordneten Abbildungsbedingungen zu bestimmen.

2. Verfahren nach Anspruch 1, bei welchem basierend auf den zumindest zum Teil bestimmten Abbildungsbedingungen und basierend auf Informationen, die eine Identifikation des spezifischen Teils (1) in der Datenbank (3) erlauben, eine Änderung der Abbildungsbedingungen mittels der Datenbank (3) bestimmt wird, um das spezifische Teil mit zumindest einer weiteren Abbildung (20) unter den geänderten Abbildungsbedingungen zu erfassen.

3. Verfahren nach Anspruch 2, bei welchem mit Hilfe der Datenbank die relative Lage des spezifischen Teils relativ zum Abbildungsbereich bestimmt wird, und
die Änderung der Abbildungsbedingungen basierend auf der relativen Lage bestimmt wird.

4. Verfahren nach Anspruch 2 oder 3, bei welchem weiter Art-Informationen bereitgestellt werden, die eine Art der Abbildung des spezifischen Teils bestimmen, wobei die Änderung der Abbildungsbedingungen zusätzlich basierend auf der Art-Information bestimmt wird.

5. Verfahren nach einem der Ansprüche 2 bis 4, bei welchem die Abbildungsbedingungen wiederholt geändert werden, bis festgestellt wird, dass der abgebildete Teil mit dem spezifischen Teil (1) zumindest im Rahmen einer vorgegebenen Toleranz übereinstimmt.

6. Verfahren nach einem der vorhergehenden Ansprüche, bei welchem der abgebildete Teil zusätzlich basierend auf der Annahme, dass der abgebildete Teil in der Nähe des spezifischen Teils (1) liegt, mittels der Datenbank erkannt wird.

7. Verfahren nach einem der vorhergehenden Ansprüche, bei welchem die Abbildungen zweidimensionale Abbildungen sind und mittels der Abfolge von Abbildungen ein dreidimensionales Modell des spezifischen Teils erstellt werden soll, wobei, falls ein Algorithmus zur Berechnung eines 3D-Modells des spezifischen Teils der anatomischen Struktur basierend auf den bereits getätigten Abbildungen nicht konvergiert oder das 3D-Modell eine vorgegebene Genauigkeit nicht erfüllt, eine weitere Abbildung unter einer anderen Abbildungsbedingung getätigt wird, die sich von den bisherigen Abbildungsbedingungen unterscheidet.

8. Verfahren nach Anspruch 7, bei welchem die zur Erstellung eines 3D-Modells eines spezifischen Teils verwendeten Abbildungsbedingungen gespeichert werden und insbesondere die Anzahl der erforderlichen Abbildungen, um ein 3D-Modell insbesondere mit vorgegebener Genauigkeit berechnen zu können, gespeichert wird.

9. Verfahren nach Anspruch 8, bei welchem bei der Auswahl einer weiteren Abbildungsbedingung auf diejenigen gespeicherten Abbildungsbedingungen zurückgegriffen wird, die bei der Erstellung eines 3D-Modells des spezifischen Teils eine vorgegebene Genauigkeit mit einer geringsten Anzahl von Abbildungen erzielten.

10. Verfahren nach einem der Ansprüche 7 bis 9, bei welchem die Abbildungsbedingungen von Abbildungssequenz zu Abbildungssequenz variiert werden, um die Anzahl der Abbildungen, die den Abbildungen jeweils zugeordneten Abbildungsbedingungen und/oder die Genauigkeit des 3D-Modells für jeden einer Anzahl bzw. Vielzahl von gespeicherten Teilen der anatomischen Struktur entsprechend einem Lernprozess zu optimieren.

11. Verfahren nach einem der vorhergehenden Ansprüche, bei welchem eine Patienten-Markereinrichtung (2) an der anatomischen Struktur (1) angebracht ist und/oder mit dem Patienten verbunden ist und/oder an einer mechanischen Ausrichthilfe, bezüglich der der Patient eine bestimmte Lage einnehmen soll, angebracht ist und bei welchem die Patienten-Markereinrichtung (2) von einer Marker-Detektionseinrichtung (5) und/oder einem Abbildungsgerät (6) das die Abbildungen durchführt, detektiert wird, um basierend auf dem Detektionsergebnis eine relative Lage und/oder Orientierung des Abbildungsgerätes (6) relativ zu dem Patienten und/oder dem spezifischen Teil (1) zu bestimmen, und basierend auf der relativen Lage und/oder Orientierung eine gegebene Abbildungsbedingung zu bestimmen.

12. Verfahren nach einem der vorhergehenden Ansprüche, bei welchem eine Abbildungs-Markereinrichtung (8) an einer Abbildungseinrichtung (4), die die Abbildungen durchführt, angebracht ist und durch die Marker-Detektionseinrichtung (5) detektierbar ist, um basierend auf dem Detektionsergebnis eine relative Lage und/oder Orientierung der Abbildungseinrichtung relativ zu dem Patienten und/oder dem spezifischen Teil (1) zu bestimmen, und um basierend auf der relativen Lage und/oder Orientierung eine gegebene Abbildungsbedingung zu bestimmen.

13. Programm, das, wenn es auf einen Computer läuft oder in einen Computer geladen wird, den Computer veranlasst, das Verfahren nach einem der Ansprüche 1 bis 13 durchzuführen.

14. Computerprogrammspeichermedium, auf dem das Programm nach Anspruch 14 gespeichert ist, oder Signalwelle, die Informationen trägt, die das Programm nach Anspruch 14 darstellen.

15. Vorrichtung zum Erfassen eines spezifischen Teils einer anatomischen Struktur mittels einer Abfolge von Abbildungen,
mit einem Abbildungsgerät (6), das ausgebildet ist,
einen Teil (1) einer anatomischen Struktur abzubilden; und
mit einer Datenbank (3), in der Informationen über die anatomische Struktur abgespeichert sind, mit einer Datenverarbeitungseinrichtung (3), die ausgebildet ist, die Datenbank (3) basierend auf der Abbildung abzufragen und mittels den gespeicherten Informationen zumindest einen Teil der Abbildungsbedingungen zu bestimmen,
**dadurch gekennzeichnet, dass**
die Abbildungen zweidimensionale Abbildungen sind und die Datenverarbeitungseinrichtung (3) ferner ausgebildet ist, mittels der Abfolge von Abbildungen ein dreidimensionales Modell des spezifischen Teils zu erstellen, wobei die Datenbank (3) für verschiedene gespeicherte Teile der anatomischen Struktur verschiedene geeignete Abbildungsbedingungen umfasst, die zur Erstellung eines dreidimensionalen Modells basierend auf den unter den geeigneten Abbildungsbedingungen erstellten zweidimensionalen Abbildungen geeignet sind, und
die Datenverarbeitungseinrichtung (3) ausgebildet ist, mit Hilfe der Datenbank (3) zu bestimmen, welche von den gespeicherten Teilen zu dem spezifischen Teil (1) passt, um die geeignete Abbildungsbedingung für eine erste und/oder nächste Abbildung basierend auf dem als passend bestimmten gespeicherten Teil und den diesen zugeordneten Abbildungsbedingungen zu bestimmen.

16. Vorrichtung nach Anspruch 15,
mit einer Eingabeeinrichtung zum Eingeben von Bestimmungs-Information, die das spezifische Teil bestimmt,
wobei die Datenverarbeitungseinrichtung (3) ausgebildet ist,
basierend auf den zumindest zum Teil bestimmten Abbildungsbedingungen und basierend auf Informationen, die eine Identifikation des spezifischen Teils in der Datenbank (3) erlauben, eine Änderung der Abbildungsbedingungen mittels den Daten der Datenbank (3) zu bestimmen, um so das spezifische Teil mit zumindest einer weiteren Abbildung unter den geänderten Abbildungsbedingungen zu erfassen.

## Claims

1. A method for capturing a specific part (1) of an anatomical structure by means of a series of images, comprising the following steps:
a) an image (10) of an imaged part of the anatomical structure is produced under imaging conditions;
b) a database (3), in which information concerning the anatomical structure is stored, is polled on the basis of the image (10), in order to determine at least some of the imaging conditions by means of the stored information;
**characterised in that**
the images are two-dimensional images, and a three-dimensional model of the specific part is produced by means of the series of images, wherein the database (3) includes different suitable imaging conditions for different stored parts of the anatomical structure, which are suitable for producing a three-dimensional model on the basis of the two-dimensional images produced under the suitable imaging conditions, and
with the aid of the database (3), it is determined which of the stored parts matches the specific part (I), in order to determine the suitable imaging condition for a first and/or next image on the basis of the stored part which has been determined to match and on the basis of the imaging conditions associated with it.

2. The method according to claim 1, wherein on the basis of the at least partly determined imaging conditions and on the basis of information which allows the specific part (I) to be identified in the database (3), a change to the imaging conditions is determined by means of the database (3), in order for the specific part to be captured by at least one subsequent image (20), under the changed imaging conditions.

3. The method according to claim 2, wherein the relative location of the specific part relative to the imaging range is determined with the aid of the database, and
the change to the imaging conditions is determined on the basis of the relative location.

4. The method according to claim 2 or 3, wherein type information is also provided which determines the type of imaging for the specific part, wherein the change to the imaging conditions is additionally determined on the basis of the type information.

5. The method according to any one of claims 2 to 4, wherein the imaging conditions are repeatedly changed until it is established that the imaged part corresponds to the specific part (1) at least within the bounds of a predetermined tolerance.

6. The method according to any one of the preceding claims, wherein the imaged part is additionally identified by means of the database on the basis of the assumption that the imaged part is lying in the vicinity of the specific part (1).

7. The method according to any one of the preceding claims, wherein the images are two-dimensional images, and a three-dimensional model of the specific part is to be produced by means of the series of images, wherein if an algorithm for calculating a 3D model of the specific part of the anatomical structure on the basis of the images already taken does not converge or the 3D model does not meet a predetermined accuracy, another image is taken under a different imaging condition which differs from the imaging conditions hitherto.

8. The method according to claim 7, wherein the imaging conditions used to produce a 3D model of a specific part are stored, and in particular the number of images required in order to be able to calculate a 3D model, in particular to the predetermined accuracy, is stored.

9. The method according to claim 8, wherein when selecting a subsequent imaging condition, reference is made to the stored imaging conditions which obtained a predetermined accuracy using a lowest number of images when producing a 3D model of the specific part.

10. The method according to any one of claims 7 to 9, wherein the imaging conditions are varied from image sequence to image sequence, in order to optimise the number of images, the imaging conditions associated with the respective images and/or the accuracy of the 3D model for each of a number of stored parts of the anatomical structure, in accordance with a learning process.

11. The method according to any one of the preceding claims, wherein a patient marker means (2) is attached to the anatomical structure (1) and/or is connected to the patient and/or is attached to a mechanical orientation aid, with respect to which the patient is to assume a particular location, and wherein the patient marker means (2) is detected by a marker detection means (5) and/or an imaging apparatus (6) which takes the images, in order to determine a relative location and/or orientation of the imaging apparatus (6) relative to the patient and/or the specific part (1) on the basis of the detection result, and to determine a given imaging condition on the basis of the relative location and/or orientation.

12. The method according to any one of the preceding claims, wherein an imaging marker means (8) is attached to an imaging means (4) which takes the images, and can be detected by the marker detection means (5), in order to determine a relative location and/or orientation of the imaging means relative to the patient and/or the specific part (1) on the basis of the detection result, and to determine a given imaging condition on the basis of the relative location and/or orientation.

13. A program which, when it is running on a computer or is loaded onto a computer, causes the computer to perform the method according to any one of claims 1 to 12.

14. A computer program storage medium, in which the program according to claim 13 is stored, or a signal wave bearing information representing the program according to claim 13.

15. A device for capturing a specific part of an anatomical structure by means of a series of images, comprising:
an imaging apparatus (6) designed to image a part (1) of an anatomical structure;
a database (3) in which information is stored concerning the anatomical structure; and
a data processing means (3) designed to poll the database (3) on the basis of the image and to determine at least some of the imaging conditions by means of the stored information,
**characterised in that**
the images are two-dimensional images, and the data processing means (3) is also designed to produce a three-dimensional model of the specific part by means of the series of images, wherein the database (3) includes different suitable imaging conditions for different stored parts of the anatomical structure, which are suitable for producing a three-dimensional model on the basis of the two-dimensional images produced under the suitable imaging conditions, and
the data processing means (3) is designed to determine, with the aid of the database (3), which of the stored parts matches the specific part (1), in order to determine the suitable imaging condition for a first and/or next image on the basis of the stored part which has been determined to match and on the basis of the imaging conditions associated with it.

16. The device according to claim 15, comprising an input means for inputting determination information which determines the specific part, wherein the data processing means (3) is designed to determine, on the basis of the at least partly determined imaging conditions and on the basis of information which allows the specific part to be identified in the database (3), a change to the imaging conditions by means of the data in the database (3), so as to capture the specific part by at least one subsequent image, under the changed imaging conditions.

## Revendications

1. Procédé pour capturer une partie spécifique (1) d'une structure anatomique au moyen d'une série d'images,
comportant les étapes suivantes consistant à :
a) générer une image (10) d'une partie reproduite en image de la structure anatomique dans des conditions d'imagerie,
b) sur la base de l'image (10), interroger une base de données (3) dans laquelle sont mémorisées des informations concernant la structure anatomique, afin de déterminer au moins une partie des conditions d'imagerie au moyen des informations mémorisées,
**caractérisé en ce que**
les images sont des images bidimensionnelles et un modèle tridimensionnel de la partie spécifique est généré au moyen de la série d'images, dans lequel la base de données (3) inclut différentes conditions d'imagerie adaptées pour différentes parties mémorisées de la structure anatomique, lesquelles conditions d'imagerie sont adaptées pour générer un modèle tridimensionnel sur la base des images bidimensionnelles générées dans les conditions d'imagerie adaptées,
la base de données (3) est utilisée pour déterminer laquelle des parties mémorisées correspond à la partie spécifique (1) afin de déterminer la condition d'imagerie adaptée pour une première image et/ou une image suivante sur la base de la partie mémorisée correspondante et des conditions d'imagerie associées à celle-ci.

2. Procédé selon la revendication 1, dans lequel sur la base des conditions d'imagerie déterminées au moins en partie et sur la base d'informations permettant une identification de la partie spécifique (1) dans la base de données (3), un changement des conditions d'imagerie est déterminé au moyen de la base de données (3) afin de capturer la partie spécifique avec au moins une image supplémentaire (20) dans les conditions d'imagerie changées.

3. Procédé selon la revendication 2, dans lequel à l'aide de la base de données, la position relative de la partie spécifique est déterminée par rapport à la région d'image, et le changement des conditions d'imagerie est déterminé sur la base de la position relative.

4. Procédé selon la revendication 2 ou 3, dans lequel des informations de type supplémentaires sont déterminées, lesquelles informations déterminent un type d'image de la partie spécifique, dans lequel le changement des conditions d'imagerie est également déterminé sur la base des informations de type.

5. Procédé selon l'une quelconque des revendications 2 à 4, dans lequel les conditions d'imagerie sont de nouveau changées jusqu'à ce qu'il soit établi que la partie représentée en image correspond à la partie spécifique (1) au moins dans les limites d'une tolérance prédéfinie.

6. Procédé selon l'une quelconque des revendications précédentes, dans lequel la partie représentée en image est également reconnue au moyen de la base de données, en prenant pour hypothèse de base que la partie représentée en image se situe à proximité de la partie spécifique (1).

7. Procédé selon l'une quelconque des revendications précédentes, dans lequel les images sont des images bidimensionnelles et un modèle tridimensionnel de la partie spécifique doit être généré au moyen de la série d'images, dans lequel, si un algorithme de calcul d'un modèle 3D de la partie spécifique de la structure anatomique ne converge pas sur la base des images déjà obtenues ou si le modèle 3D ne respecte pas une précision prédéfinie, une image supplémentaire est obtenue dans une autre condition d'imagerie qui diffère des anciennes conditions d'imagerie.

8. Procédé selon la revendication 7, dans lequel les conditions d'imagerie utilisées pour générer un modèle 3D d'une partie spécifique sont mémorisées, et le nombre des images requises pour pouvoir calculer un modèle 3D, en particulier avec une précision prédéfinie, est en particulier mémorisé.

9. Procédé selon la revendication 8, dans lequel pour la sélection d'une condition d'imagerie supplémentaire, une référence est faite aux conditions d'imagerie mémorisées qui ont permis d'obtenir le plus petit nombre d'images lors de la génération d'un modèle 3D de la partie spécifique avec une précision prédéfinie.

10. Procédé selon l'une quelconque des revendications 7 à 9, dans lequel les conditions d'imagerie sont changées de séquence d'images en séquence d'images afin d'optimiser le nombre d'images, les conditions d'imagerie respectivement associées aux images et/ou la précision du modèle 3D pour chaque nombre ou pluralité de parties mémorisées de la structure anatomique, conformément à un processus d'apprentissage.

11. Procédé selon l'une quelconque des revendications précédentes, dans lequel un dispositif de marquage de patient (2) est appliqué sur la structure anatomique (1) et/ou est relié au patient et/ou est appliqué sur une aide mécanique à l'orientation, par rapport à une position déterminée que le patient doit prendre, et dans lequel le dispositif de marquage de patient (2) est détecté par un dispositif de détection de marqueur (5) et/ou un dispositif d'imagerie (6) qui obtient les images, afin de déterminer, sur la base du résultat de la détection, une position et/ou une orientation relative de l'appareil d'imagerie (6) par rapport au patient et/ou à la partie spécifique (1), et pour déterminer, sur la base de la position et/ou de l'orientation relative, une condition d'imagerie donnée.

12. Procédé selon l'une quelconque des revendications précédentes, dans lequel un dispositif de marquage d'image (8) est appliqué sur un dispositif d'imagerie (6) qui obtient les images, et peut être détecté par le dispositif de détection de marqueur (5) afin de déterminer, sur la base du résultat de la détection, une position et/ou une orientation relative du dispositif d'imagerie par rapport au patient et/ou à la partie spécifique (1), et pour déterminer, sur la base de la position et/ou de l'orientation relative, une condition d'imagerie donnée.

13. Programme qui, lorsqu'il s'exécute sur un ordinateur ou lorsqu'il est chargé dans un ordinateur, amène l'ordinateur à mettre en oeuvre le procédé selon l'une quelconque des revendications 1 à 12.

14. Support de mémorisation de programme informatique sur lequel est mémorisé le programme selon la revendication 13, ou onde de signal transportant des informations qui constituent le programme selon la revendication 13.

15. Dispositif pour capturer une partie spécifique d'une structure anatomique au moyen d'une série d'images, comportant :
un appareil d'imagerie (6) qui est conçu pour représenter en image une partie (1) d'une structure anatomique, et
une base de données (3) dans laquelle sont mémorisées des informations concernant la structure anatomique,
un dispositif de traitement de données (3) qui est conçu pour interroger la base de données (3) sur la base de l'image et pour déterminer, au moyen des informations mémorisées, au moins une partie des conditions d'imagerie,
**caractérisé en ce que** les images sont des images bidimensionnelles et le dispositif de traitement de données (3) est en outre conçu pour générer un modèle tridimensionnel de la partie spécifique au moyen de la série d'images, dans lequel la base de données (3) inclut différentes conditions d'imagerie adaptées pour différentes parties mémorisées de la structure anatomique, lesquelles conditions d'imagerie sont adaptées pour générer un modèle tridimensionnel sur la base des images bidimensionnelles générées dans les conditions d'imagerie adaptées, et
le dispositif de traitement de données (3) est conçu pour déterminer, à l'aide de la base de données (3), laquelle des parties mémorisées correspond à la partie spécifique (1) afin de déterminer la condition d'imagerie adaptée pour une première image et/ou une image suivante sur la base de la partie mémorisée déterminée comme étant correspondante, et les conditions d'imagerie associées à celle-ci.

16. Dispositif selon la revendication 15, comportant un dispositif d'entrée pour entrer des informations de détermination qui déterminent la partie spécifique,
dans lequel le dispositif de traitement de données (3) est conçu pour déterminer, sur la base des conditions d'imagerie déterminées au moins en partie et sur la base d'informations permettant une identification de la partie spécifique dans la base de données (3), un changement des conditions d'imagerie au moyen des données de la base de données (3) afin de capturer la partie spécifique avec au moins une image supplémentaire dans les conditions d'imagerie changées.
